(19) Europäisches Patentamt European Patent Office Office européen des brevets

(11) **EP 1 694 140 B1**

(12) **EUROPEAN PATENT SPECIFICATION**

(45) Date of publication and mention of the grant of the patent:
**05.08.2009 Bulletin 2009/32**

(21) Application number: **04820397.0**

(22) Date of filing: **19.11.2004**

(51) Int Cl.:
*A23L 1/305* [(2006.01)]    *A23J 3/34* [(2006.01)]
*A61K 38/06* [(2006.01)]    *A61K 38/04* [(2006.01)]

(86) International application number:
**PCT/EP2004/013223**

(87) International publication number:
**WO 2005/058070 (30.06.2005 Gazette 2005/26)**

(54) **PEPTIDES HAVING AN ACE INHIBITING EFFECT**

PEPTIDE MIT ACE-HEMMWIRKUNG

PEPTIDES PRESENTANT UN EFFET INHIBITEUR DE L'ECA

(84) Designated Contracting States:
**AT BE BG CH CY CZ DE DK EE ES FI FR GB GR HU IE IS IT LI LU MC NL PL PT RO SE SI SK TR**

(30) Priority: **15.12.2003 EP 03079075**

(43) Date of publication of application:
**30.08.2006 Bulletin 2006/35**

(73) Proprietors:
• **Unilever N.V.**
**3013 AL Rotterdam (NL)**
Designated Contracting States:
**SK TR AT BE BG CH CZ DE DK EE ES FI FR GR HU IS IT LI LU MC NL PL PT RO SE SI**
• **Unilever PLC**
**London**
**EC4Y 0DY (GB)**
Designated Contracting States:
**CY GB IE**

(72) Inventors:
• **ABRAHAMSE, S. L.,**
**Unilever R&D Vlaardingen**
**NL-3133 AT Vlaardingen (NL)**
• **DRAAISMA, R. B.,**
**Unilever R&D Vlaardingen**
**NL-3133 AT Vlaardingen (NL)**
• **SCHALK, J.,**
**Unilever R & D Vlaardingen**
**NL-3133 AT Vlaardingen (NL)**
• **VAN PLATERINK, C. J.,**
**Unilever R&D Vlaardingen**
**NL-3133 AT Vlaardingen (NL)**

(74) Representative: **Joppe, Hermina Laura Petronella et al**
**Unilever Patent Group**
**Olivier van Noortlaan 120**
**3133 AT Vlaardingen (NL)**

(56) References cited:
EP-A- 1 231 279       WO-A-01/68114
WO-A-01/85984       US-A- 4 719 200
US-A- 5 356 637

• **TAKANO T: "Milk derived peptides and hypertension reduction" INTERNATIONAL DAIRY JOURNAL, ELSEVIER APPLIED SCIENCE, BARKING,, GB, 1998, pages 375-381, XP002236259 ISSN: 0958-6946**
• **PATENT ABSTRACTS OF JAPAN vol. 1998, no. 13, 30 November 1998 (1998-11-30) & JP 10 212245 A (AGENCY OF IND SCIENCE &TECHNOL; SHOWA SANGYO CO LTD), 11 August 1998 (1998-08-11)**
• **DATABASE FSTA [Online] INTERNATIONAL FOOD INFORMATION SERVICE (IFIS), FRANFURT/MAIN, DE; 2004, KWANG-SEOK OH ET AL: "Peptide inhibitors for angiotensin I converting enzyme from corn gluten digests." XP002279625 Database accession no. 2004-00-m0121 & KOREAN JOURNAL OF MICROBIOLOGY AND BIOTECHNOLOGY 31 (1) 51-56 2003 CORRESPONDENCE (REPRINT) ADDRESS, HA-CHIN SUNG, GRADUATE SCH. OF BIOTECH., KOREA UNIV., SEOUL 136-701, KOREA. TEL. 82-2-3290-3418. FAX 82-2-923-9923. E-MAIL HCSUNG(A)KOREA.AC.KR, 2003,**

- PIHLANTO-LEPPALA A ET AL: "Angiotensin I-converting enzyme inhibitory properties of whey protein digests: concentration and characterization of active peptides" JOURNAL DAIRY RESEARCH, CAMBRIDGE, GB, vol. 67, no. 1, 2000, pages 53-64, XP008022966
- DATABASE WPI Section Ch, Week 200382 Derwent Publications Ltd., London, GB; Class B04, AN 2003-884296 XP002318485 & JP 2003 267994 A (SUETSUNA A) 25 September 2003 (2003-09-25)
- DATABASE MEDLINE [Online] US NATIONAL LIBRARY OF MEDICINE (NLM), BETHESDA, MD, US; 15 November 1978 (1978-11-15), DORER F E ET AL: "Studies on the hydrolysis of bradykinin by angiotensin-converting enzyme (kininase II)." XP002279624 Database accession no. NLM214326 & EXPERIENTIA. SWITZERLAND 15 NOV 1978, vol. 34, no. 11, 15 November 1978 (1978-11-15), page 1436, ISSN: 0014-4754

**Description**

**Field of the invention**

The invention relates to certain peptides for the preparation of an angiotensin-converting enzyme (ACE) inhibitor. The invention further relates to food products suitable for ACE inhibition and to a process for preparing such food products.

**Background to the invention**

Hypertension or high blood pressure is considered to be one of the main risk factors for Cardio Vascular Diseases (CVD). One of the mechanisms which regulates blood pressure is the renin-angiotensin system. This is a cascade of reactions leading to the formation of angiotensin II, which has a strong vasoconstrictive and hence blood pressure increasing effect. Inhibition of one of the key enzymes in this cascade: Angiotensin I Converting Enzyme (ACE) reduces formation of angiotensin II and thus has a blood pressure lowering effect. Long term human intervention studies have shown regular intake of low amounts of ACE inhibitors reduces CVD by 25% (Gerstein et al. (2000), The Lancet 355, 253-259).
ACE-inhibitors in food products are well known. Such food products have for instance been prepared by fermentation of milk or milk products. In a placebo-controlled study, the blood pressure lowering effect of VPP and IPP in sour milk was shown in hypertensive humans (Hata, Y et al. (1996), American Journal of Clinical Nutrition 64, 767-771). WO 01/68114, JP 10212245, FSTA AN 2004-00-M0121 and EP 1231279 refer to tripeptides HPP, LPP, GPP and VPP and IPP respectively, as ACE inhibitors.
A commercially available fermented milk product, which claims to be "suitable for those with mild hypertension" is Calpis sour milk, fermented with *Lactobacillus helveticus,* produced by Calpis Food Industry, Japan. Another commercially available fermented milk product is Evolus produced by Valio, Finland, which claims to be the first European functional food to help lower blood pressure'. These fermented milk products are fermented with *Lactobacillus helveticus* (*Lb. helveticus*) strains. The products contain bio-active peptides (VPP and IPP) responsible for *in vitro* ACE inhibition, which are produced by proteolysis of caseins.
Another possibility identified in the prior art is preparation of ACE-inhibiting food products by enzymatic hydrolysis of milk proteins. WO 01/85984 describes the preparation of an ACE-suppressing composition by hydrolysis of casein isolate using the enzyme trypsin.

**Summary of the invention**

It is an object of the invention to provide a food product suitable for ACE inhibition. It is another object to provide such food products having blood pressure lowering effect. It is still a further object to provide a food product having a high concentration of ACE-inhibitor. One or more of these objects is attained according to the invention by the use of the tripeptide XPP, wherein X= C, M, S, T, or K and/or the salts thereof for the preparation of an angiotensin-converting enzyme inhibitor.

**Detailed description of the invention**

The common one letter code is used herein as ordinarily used to describe amino-acids.
The weight percentages herein will be expressed:relative to the total weight, unless otherwise indicated.
Enzyme is herein understood to also include a mixture of more than one enzymes.
According to the invention, we have found that the tripeptide XPP according to the invention is relatively stable after human consumption and that the peptide XPP according to the invention is an effective angiotensin-converting enzyme inhibitor. Preferably the angiotensin-converting enzyme inhibitor is a functional food product.
In XPP, X= C, M, S, T, or K. Preferably X= C, M, S, or T, more preferably X= M, S, or T.
The invention provides a food product suitable for angiotensin-converting enzyme inhibition and lowering of blood pressure in humans comprising an amount 1 mg/g or more of XPP, wherein X= C, M, S, T, or K, preferably 25mg/g or more of XPP, more preferably the food product comprises an amount of 50 mg/g or more, even more preferably 100 mg/g or more XPP, wherein X= C, M, S, T, or K, preferably X= C, M, S, or T, more preferably X= M, S, or T.
Food products according to the invention are defined as products, suitable for human consumption, in which an XPP according to the invention was used as an ingredient in an effective amount, such that a noticeable ACE-inhibitory effect is obtained.
Though not wishing to be bound to theory herein, it is believed that the tripeptides according to the invention are good ACE-inhibitors, since they are:

- relatively small in size [length between 2 and 10 amino acids]
- neutral or positively charged
- not composed of negatively charged residues
- composed of hydrophobic, branched amino acids
- have a C-terminal Proline residue

Further it is believed that to the presence of two successive Pro-residues, the release of the last two residues by ACE in the case of XPP is significantly hindered due to steric constraints.

Additionally it is believed that the presence or the C-terminal PP (-Pro-Pro) sequence renders the peptide(s) more stable towards human digestive enzymes, peptidases associated with brush-border membrane of the human intestinal epithelium cells and peptidases in the human blood circulation. Therefore, it is believed that the consumption of a food product containing the peptide(s) will result in the effective absorption of the peptide(s) in the human body, which will induce ACE-inhibition and lowering of blood pressure.

Summarising these observations it can be deduced that any peptide that that is not too long and composed of hydrophobic amino acids, neutral or positively charged and bearing a C-terminal Pro [or-Pro-Pro] sequence is intrinsically a good inhibitor for ACE.

In general the results are in agreement with the observations, the best ACE-inhibiting XPP-peptides are composed of neutral and hydrophobic amino acids. The reason that large hydrophobic residues like Trp, Tyr and Phe are not included resides from the fact that - most likely - these residues, in combination with the two Pro residues, are too bulky for a proper fit in the active site of the enzyme.

The reason that CPP is included derives from the fact that ACE is a Zn-containing enzyme and interaction of the substrate with the Zn-atom is essential for binding between substrate and enzyme, the presence of the free sulphydryl-group of Cys favours this interaction.

The following proteins (and their precursors) have, for example, been identified to contain mentioned XPP sequences within the primary structure of their constitutional proteins and their sources:

CPP:    collagen [chicken], troponin [chicken], thyroglobin [bovine]
MPP:    albumin [garden pea, mung bean], myosin [bovine, chicken, pig, yeast], zein [maize]
SPP:    collagen [chicken], casein [bovine], legumin [garden pea, Pea Sativum].
TPP:    collagen [chicken], glutenin [wheat], cruceferin [rape], legumin [cotton], myosin [yeast], zein [maize]
TPP:    collagen [chicken], glutenin [what], cruceferin [rape], legumin [cotton], myosin [yeast], zein [maize]
KPP:    myosin [pig, chicken, yeast], casein [pig]

These protein materials may, for instance be used as a substrate, from which the peptides according to the invention may be liberated. The skilled person will know to use known fermentation or known enzyme treatment, for instance enzymatic hydrolysis to achieve this.

Through optimisation of the fermentation or hydrolysis conditions, the production of the biologically active molecule XPP according to the invention may be maximised. The skilled person trying to maximise the production will know how to adjust the process parameters, such as hydrolysis time, hydrolysis temperature, enzyme type and concentration etc.

Preferably, in the production of the XPP, the molar yield of XPP is high. The molar yield of XPP is defined as the molar amount of XPP produced divided by the molar amount of XPP fragments in the total mass of protein present in the starting material prior to hydrolysis.

The enzyme used in enzymatic hydrolysis may be any enzyme that is able to effectively hydrolyse a substrate containing XPP, resulting in the liberation of one or more XPP, wherein X= C, M, S, T, or K.

The enzyme treatment may be done in a conventional manner. It involves adding enzyme (or a mixture of enzymes) to the substrate and maintaining the resulting reaction mixture under controlled conditions suitable for conducting the enzymatic hydrolysis. The conditions to be controlled include temperature, pH, reaction time and enzyme concentration. The preferred temperature of the reaction mixture is 40-60 degrees C, more preferred 45-55 degrees C and most preferred about 50 degrees C. The pH of the reaction mixture is preferably 5 to 9, more preferably 6-8 and most preferably about 7. The enzyme concentration is 2-10 wt.% based on the total weight of the reaction mixture, more preferably 3-10 and most preferably 4-6 wt%. The reaction time (hydrolysis time) is preferably 2-50 hours, more preferably 2- 10 hours and most preferably 4-8 hours.

In another preferred embodiment the hydrolysis step may be substituted by a fermentation step with microorganism that will cause hydrolysis of the substrate.

The materials in the fermentor and substrate may be mixed in conventional way, in order to achieve a homogeneous fermentation medium.

The fermentation advantageously may be performed at 25 to 50° C and preferably 35 to 45° C, for 3 to 80 hours and preferably 6 to 25 hours.

Advantageously, after enzyme treatment and optional fermentation, several additional process steps may be executed.

Preferably a process for the preparation of a food product comprising XPP, wherein X= C, M, S, T, or K, involving the following steps is.used:

a)enzymatic hydrolysis of a substrate comprising protein having the sequence XPP, resulting in a hydrolysed protein product;

b)separation from the hydrolysed protein product of a fraction rich in tripeptide;

c)drying the fraction from step b) to obtain a solid rich in tripeptide XPP;

d)using the solid prepared in step c) as an ingredient in the preparation of the food product.

After the enzymatic hydrolysis step a), therefore there may be an optional separation step or concentration step. This step may be executed in any way known to the skilled person, e.g. by filtration, centrifugation or chromatography and combinations thereof. Preferably the separation step is executed using an ultrafiltration (UF) and/or nanofiltration (NF) techniques. The pore size of the membranes used in the filtration step, as well as the charge of the membrane may be used to control the separation of the tripeptide XPP. The fractionation of protein hydrolysates using charged UF/NF membranes is described in Y. Poilot et al, Journal of Membrane Science 158 (1999) 105-114. Electrodialysis is for instance described in WO00/42066. The product of such separation step is herein called the ACE-fraction.

Optionally the hydrolysis product may be dried, to obtain a solid rich in tripeptide XPP, wherein X= C, M, S, T, or K. This step may be done in a conventional way, e.g. by spray drying or freeze drying.

The dried product prepared in is hereafter designated as ACE-solid. The ACE-fraction and/or the ACE-solid may advantageously be used as an ingredient in a food product.

The food product according to the invention or food products derived therefrom may be pasteurised or sterilised.

The food products according to the invention may be of any food type. They may comprise common food ingredients in addition to the food product, such as flavour, sugar, fruits, minerals, vitamins, stabilisers, thickeners, etc. in appropriate amounts.

Preferably, the food product comprises 50-200 mmol/kg $K^+$ and/or 15-60 mmol/kg $Ca^{2+}$ and/or 6-25 mmol/kg $Mg^{2+}$ more preferably, 100-150 mmol/kg $K^+$ and/or 30-50 mmol/kg $Ca^{2+}$ and/or 10-25 mmol/kg $Mg^{2+}$ and most preferably 110-135 mmol/kg $K^+$ and/or 35-45 mmol/kg $Ca^{2+}$ and/or 13-20 mmol/kg $Mg^{2+}$.

Preferably the food products are fruit juice products, dairy type products, frozen confectionary products or spreads/margarines. These preferred types of food products are described in some detail below and in the examples.

Fruit juice products

Examples of fruit juice products according to the invention are juices derived from citrus fruit like orange and grapefruit, tropical fruits, banana, peach, peer, strawberry, to which ACE-solids and/or ACE-fraction are added.

Dairy type products

Examples of dairy products according to the invention are milk, dairy spreads, cream cheese, milk type drinks and yoghurt, to which ACE-solids and/or ACE-fraction are added or in which XPP is produced during preparation of the food product. The food product may be used as such as a milk type drink. Alternatively or additionally flavour or other additives may be added. A dairy type product may also be made by adding ACE-solids and/or ACE-fraction to water or to a dairy product.

An example of a composition for a yoghurt type product is about 50-80 wt.% water, 0.1-15 wt.% ACE-solids, 0-15 wt.% whey powder, 0-15 wt.% sugar (e.g. sucrose), 0.01-1 wt.% yoghurt culture, 0-20 wt.% fruit, 0.05-5 wt.% vitamins and minerals, 0-2 wt.% flavour, 0-5 wt.% stabilizer (thickener or gelling agent).

A typical serving size for a yoghurt type product could be from 50 to 250 g, generally from 80 to 200 g.

Frozen Confectionery Products

For the purpose of the invention the term frozen confectionery product includes milk containing frozen confections such as ice-cream, frozen yoghurt, sherbet, sorbet, ice milk and frozen custard, water-ices, granitas and frozen fruit purees.

Preferably the level of solids in the frozen confection (e.g. sugar, fat, flavouring etc) is more than 3: wt.%, more preferred from 10 to 70 wt.%, for example 40 to 70 wt.%.

Ice cream will typically comprise 0 to 20 wt.% of fat, 0.1 to 20 wt.% ACE-solids, sweeteners, 0 to 10 wt.% of non-fat milk components and optional components such as emulsifiers, stabilisers, preservatives, flavouring ingredients, vitamins, minerals, etc, the balance being water. Typically ice cream will be aerated e.g. to an overrun of 20 to 400 %, more specific 40 to 200 % and frozen to a temperature of from -2 to -200 °C, more specific -10 to -30 °C. Ice cream normally comprises calcium at a level of about 0.1 wt%.

Other food products

Other food product according to the invention can be prepared by the skilled person based on common general knowledge, using hydrolysed protein as a base material for food or using derived products, such as ACE-solids as an ingredient in suitable amounts. Examples of such food products are baked goods, dairy type foods, snacks, etc.

Advantageously the food product is an oil and water containing emulsion, for instance a spread. Oil and water emulsion is herein defined as an emulsion comprising oil and water and includes oil in water (O/W) emulsions and water in oil emulsions (W/O) and more complex emulsions for instance water-in-oil-in-water (W/O/W/O/W) emulsions. Oil is herein defined as including fat. Preferably the food product is a spread, frozen confection, or sauce. Preferably a spread according to the invention comprises 30-90 wt.% vegetable oil. Advantageously a spread has a pH of 4.2-6.0.

**Examples**

*Example 1*

A large number of peptides were screened for their ACE-inhibiting effects ($IC_{50}$ values were measured). In addition all peptides were screened for stability during exposure to human serum, HUVEC, Caco-2 cells and gastrointestinal enzymes as described below.

The materials and methods used in Example 1 are described below.

*Cell culture*

Caco-2 cells were obtained from American Type Culture Collection (ATCC) and used in experiments at passage 30-40. Cells were cultured in 75 cm$^2$ culture flasks (Corning Costar, Badhoevedorp, The Netherlands). The culture medium consisted of DMEM (high glucose, with L-glutamine) supplemented with 20% (v/v) foetal bovine serum, 1% (v/v) penicillin/streptomycin solution and 1% (v/v) NEAA. Cells were maintained at 37° C in a humidified atmosphere of 5% $CO_2$ in air. For stability experiments, cells were seeded on 12 well cell culture plates (Costar, Badhoevedorp, The Netherlands) and cultured for at least 21 drays.

HUVEC Cells

HUVEC cells were obtained from (Cambrex Bio Science, Verviers, Belgium) and used in experiments at passage 1-5. Cells were cultured in 75 cm$^2$ culture flasks (Corning Costar, Badhoevedorp, The Netherlands). The culture medium (Cambrex) consisted of EBM-2 supplemented with 2% (v/v) foetal bovine serum, 0.04 % (v/v) Hydrocortisone, 0.4 % (v/v) human Fibroblast Growth Factor Basic with heparin, 0.1 % (v/v) Vascular Endothelial Growth Factor, 0.1 % (v/v) human recombinant Insulin-like Growth Factor, 0.1 % (v/v) Ascorbic acid, 0,1 % (v/v) human recombinant Epidermal Growth Factor, 0.1 % (v/v) Gentamicin, Amphotericin-B and 0.1% (v/v) Heparin. Cells were maintained at 37° C in a humidified atmosphere of 5% $CO_2$ in air. For stability experiments, cells were seeded in a 12 well plate (Corning Costar, Badhoevedorp, The Netherlands) at a density of 100.000 cells/well and cultured for 2 days.

*Test products*

Peptide mixture 1 consisted of VPP, IPP, IIAEK, ITP, VF, FY, KVLPVP, and HLPLP. Mixture 2 consisted of VAP, GPR, CPP, MPP, SPP, TPP, PIP, and PLP. The synthetic peptides were either ordered from Bachem (dipeptides KPP and GPR) or from the University of Utrecht (Dr. M. Egmond).:

The peptides were dissolved separately in a concentration of 5 mg in 50 μl milliQ water or DMSO. For some peptides the concentration was corrected for purity (ITP 60%, KVLPVP 85%). The remaining peptides were >95% pure. All peptides were dissolved in milli Q water except for the dipeptides. FY was dissolved in DMSO. Since FY was still not soluble some HCl (final concentration 0.3M) was added. VF was directly dissolved in 0.3M HCl. To prepare mixture 1, 50 μl of each peptide were mixed together and 100 μl milli Q water was added, yielding a 500 μl solution containing 5

mg of each peptide (in 0.06M HCl and 10% DMSO). Mixture 2 was prepared in the same way, with the exception of the addition of an extra 50 µl milli Q water. The final composition of mixture 2 was also 10mg/ml of each peptide.

Stability tests of peptides

*Stability tests with serum, HUVEC and Caco-2 cells*

Peptide mixtures were added to human serum (Cambrex Bio Science, Verviers, Belgium) at a final concentration of 0,1 1 and 10 µg peptide/L. Serum containing the peptide-mixtures were incubated for 0, 1, 2, 5, 10, 20, 60, 120, 240 and 360 minutes at 37° C. Samples of 1.5 ml were collected in eppendorf vials at each of the time points. Immediately thereafter, 30 µl of 10% (v/v) trifluoro acetic acid (TFA) and 5 µl of a 10 µg UC13-IPP/10 ml were added to the samples. Samples were subsequently incubated for 5 min at 100° C and then centrifugated in an IEC Micromax RF centrifuge (Boom BV Meppel) at 10.000 rpm for 20 minutes. The supernatant was collected in eppendorf vials and stored at -20°C for further analysis. Experiments were performed in triplicate.

Cells were cultured in 12-well plates. Peptide mixtures were added to culture medium without foetal bovine serum, but containing 20 % (v/v) solid phase extracted foetal bovine serum. For HUVEC, 400 µl medium containing 0.1, 1 and 10 µg/ml peptide was added to a well of the culture plate. Three times 130 µl medium was collected at 0, 5, 10, 20, 30, 60, 90 and 120 min of incubation. For Caco-2 cells, 330 µl medium containing 0.1, 1 and 10 µg/ml peptide was added to each well of the culture plate. Three times 110 µl medium was collected at 0, 5, 10, 20, 30, 60, 90, 180 and 360 min of incubation. Experiments were performed in triplicate and all samples were directly placed on dry-ice and stored at - 20°C. For peptide quantification measurement, TFA and UC13-IPP (final concentrations of 0.2% (v/v) and 60 ng/ml, respectively) were added to the samples. Wells without cells served as controls.

*ACE-inhibition assay 1: Enzyme based Assay (EBA)*

ACE and a synthetic substrate (Abz-FRK-(Dnp)P-OH) were used in the ACE-inhibition assay performed in white optiplate-96 microplates (Packard Bioscience). The substrate was a kind gift of Adriana K. Carmona (Dept. of Biophysics, Escola Paulista de Medivina, Universidae Federal de Sao Paulo, Brazil). Stock solutions of Abz-FRK-(Dnp) P-OH were prepared in DMSO. The concentration was measured spectrophotometrically using the molar extinction coefficient $\varepsilon_{365}$ = 17300 $M^{-1}cm^{-1}$. The assay buffer composition was 100 mM tris/HCl buffer pH 7.0 containing 100 mM NaCl. Forty µl sample solution in assay buffer was added to each well. In case of the controls only buffer was added. The samples were measured in threefold in a fluorophotometer (Fluostar, BMG labtechnologies). The device first dispersed 150µl substrate (3.75 µM in assay buffer) and subsequently added 20µl of ACE (0.00625 Units/ml) to each well. The ACE activity was measured for 10 cycles (about 10 minutes) by measuring the fluorescence at $\lambda$ex = 320nm and $\lambda$em = 420nm. The raw data was converted to the slope/sec and the ACE inhibition activity was calculated using the equation below.

$$\text{ACE inhibition (\%)} = [1 - (S_{mean} - B_{mean}) / (C_{mean} - B_{mean})] *100$$

Where

S_{mean} = average result of sample (peptide + substrate + ACE)
B_{mean} = average result of background (substrate)
C_{mean} = average result of control (ACE + substrate)

*ACE-inhibition assay 2: Modified Matsui assay*

This ACE inhibition activity was assayed according to the method of Matsui et al. (Matsui, T. et al. (1992) Biosci. Biotech. Biochem. 56: 517-518) with the modifications described below.

Table 1: procedure for Matsui ACE inhibition assay. The components were added in a 1.5-ml tube with a final volume of 120 $\mu$l.

| Component | Control 1 ($\mu$l) | Control 2 ($\mu$l) | Sample 1 ($\mu$l) | Sample 2 ($\mu$l) |
|---|---|---|---|---|
| HHL (3 mM) | 75 | 75 | 75 | 75 |
| H$_2$O | 25 | 45 | - | 20 |
| Sample/inhibit or | - | - | 25 | 25 |
| ACE (0.1 U/ml) | 20 | - | 20 | - |

For each sample 75 $\mu$l 3 mM hippuryl histidine leucine (Hip-His-Leu, Sigma chemicals Co.; the chemical was dissolved in 250 mM Borate containing 200 mM NaCl, pH 8.3); 20 $\mu$l 0.1 U/ml ACE (obtained at Sigma) or H$_2$O and 25 $\mu$l sample or H$_2$O were mixed (see Table 1). The mixtures were incubated at 37°C and stopped after 30 min by adding 125 $\mu$l 0.5 M HCl. Subsequently, 225 $\mu$l bicine/NaOH solution (1 M NaOH : 0.25 M bicine (4:6)) was added, followed by 25 $\mu$l 0.1 M TNBS (2,4,6-Trinitrobenzenesulfonic acid, Fluka, Switzerland; in 0.1 M Na$_2$HPO$_4$). After incubation for 20 min. at 37°C, 4 ml 4 mM Na$_2$SO$_3$ in 0.2 M NaH$_2$PO$_4$ was added and the absorbance at 416 nm was measured with UV/Vis spectrophotometer (Shimadzu UV-1601 with a CPS controller, Netherlands).

The amount of ACE inhibition (ACEI) activity was calculated as a percentage of inhibition compared with the conversion rate of ACE in the absence of an inhibitor:

$$ACEI\ (\%) = (((C1-C2)-(S1-S2))/(C1-C2)) * 100 \qquad (1)$$

wherein

C1 = Absorbance without ACE inhibitory component (= max. ACE activity) [AU].
C2 = Absorbance without ACE inhibitory component and without ACE (background) [AU].
S1 = Absorbance in the presence of ACE and the ACE inhibitory component [AU].
S2 = Absorbance in the presence of the ACE inhibitory component, but without ACE [AU].

*Quantification of peptides using HPLC-MRM-MS*

All measurements were carried out on a Waters Quattro-II or Quattro-Ultima triple quadrupole mass spectrometer. The LC separation was carried out at 25° C on a Inertsil 5 ODS3 column, 150 x 2.1 mm, packed with 3 $\mu$m particles (Chompack) using 0-50% acetonitrile/ 0.1% (v/v) TFA. For analyses, 5 $\mu$l sample was injected onto the column. The flow rate through the column was 0.2 ml/min. The total flow after post-column makeup was 0.05 ml/min and contained a 7/3 (v/v) mixture of propionic acid and propanol-2. Gradient effluent was analysed using MRM mass spectrometry in positive-ESI mode. Capillary voltage was 4 kV. Source and nebulizer temperature were 100 °C and 250 °C, repsectively. Drying and nebuliser gas flow were 300 l/h and 17 l/h, respectively. Collision gas was argon at a pressure of 7.9 e-4 mbar. The MS-data for the analysed peptides are given in table 2.

Table 2: Description of the MS-parameters for the selected peptides.

| Peptide | Precursor ion | Cone voltage | Product ion | Mass structure | Collision energy |
|---|---|---|---|---|---|
| VPP | 312.2 | 19 | 213.1 | PP | 18 |
| IPP | 326.2 | 19 | 213.1 | PP | 18 |
| ITP | 330.2 | 16 | 116.1 | P | 11 |
| IIAEK | 573.4 | 40 | 347.2 | AEK | 26 |
| MPP | 344.2 | 19 | 213.1 | PP | 21 |
| SPP | 300.2 | 18 | 185.1 | SP | 14 |
| TPP | 314.2 | 18 | 199.1 | TP | 16 |

(continued)

| Peptide | Precursor ion | Cone voltage | Product ion Mass structure | | Collision energy |
|---|---|---|---|---|---|
| GPR | 329.2 | 23 | 175.1 | PR | 25 |
| PIP | 326.2 | 16 | 211.1 | IP | 13 |
| PLP | 326.2 | 16 | 211.1 | LP | 13 |
| VF | 265.1 | 18 | 72.0 | V | 15 |
| FY | 329.1 | 23 | 120.1 | F | 18 |
| HLPLP | 576.3 | 25 | 251.2 | HL | 20 |
| KVLPVP | 652.4 | 36 | 341.3 | KVL | 24 |
| VAP | 286.2 | 15 | 116.1 | P | 13 |

In vitro *gastrointestinal digestion*

The stability of peptides in the human gastrointestinal tract was studied by subjecting fermented or hydrolysed milk proteins to typical conditions in the stomach and small intestine. Gastric conditions were mimicked by dissolving 80 ml fermented or hydrolysed milk protein in 820 ml water contianing 2.0 g NaCl, 2.9 g pepsin and 0.45 g Amano Lipase F-AP15 from *Rhizopus oryzae.* The fluid was adjusted to pH 3.5 with HCl, stirred with a peddle (50 rpm) and kept at 37° C for 60 min. Subsequently, intestinal conditions were mimicked by adding 9 g pancreatin and 125 mg bile-extract to the simulated gastric fluid and adjusting the pH to 6.8 with NaHCO$_3$. The simulated intestinal fluid was stirred with a peddle (50 rpm) and kept at 37° C for 120 min. Samples were collected at different time points during the *in vitro* gastrointestinal digestion. After collection, samples were directly heated at 95° C for 30 min and subsequently stored at -20° C.

The results of example 1 are given in table 3.

Table 3: Stability of peptides during exposure to human serum, HUVEC, Caco-2 cells and gastrointestinal proteases (GI conditions) as determined by LC-MS.

| Peptide | Serum | HUVEC | Caco-2 | GI conditions | IC$_{50}$ Matsui ($\mu$M) | IC$_{50}$ EBA ($\mu$M) |
|---|---|---|---|---|---|---|
|  |  |  |  |  |  |  |
| VPP | +/- | + | - | + | 3 | 20 |
| IPP | +/- | + | - | + | 2 | 12 |
| ITP | - | - | - | ND | 10 | 30 |
| IIAEK | - | - | - | +/- | >20 | 20 |
| VF | +/- | - | +/- | ND | 10 | >100 |
| FY | + | - | +/- | ND | 10 | 20 |
| HLPLP | +/- | + | + | + | 17 | 15 |
| KVLPVP | - | - | - | ND | 2 | 5 |
| VAP | - | - | - | ND | 1 | 4 |
| GPR | - | + | - | ND | ND | ND |
| KPP | ND | ND | ND | ND | 50 | >50 |
| CPP | ND | ND | ND | ND | 4 | 5 |
| MPP | +/- | + | - | ND | 7 | 10 |
| SPP | +/- | + | + | ND | >50 | 75 |
| TPP | +/- | + | + | ND | 15 | 35 |
| PIP | - | +/- | - | ND | ND | >50 |

(continued)

| Peptide | Serum | HUVEC | Caco-2 | GI conditions | IC$_{50}$ Matsui ($\mu$M) | IC$_{50}$ EBA ($\mu$M) |
|---|---|---|---|---|---|---|
| PLP | - | +/- | - | ND | ND | >50 |
| Explanation of signs used in table 3:<br>+ = stable<br>- = fast degradation by cells or serum<br>+/- = slow degradation by cells or serum<br>ND = not determined | | | | | | |

Table 3 shows that all XPP's wherein X= C, M, S, T, or K are moderate to good ACE-inhibitors (low IC$_{50}$ values). Further these peptides show, compared to other peptides tested, slow degradation when subjected to human serum and they are relatively stable when subjected to HUVEC, Caco-2 and gastrointestinal enzymes.

## Claims

1. Use of the tripeptide XPP, wherein X= C, M, S, T, or K and/or salts thereof for the preparation of an angiotensin-converting enzyme inhibitor.

2. Use according to claim 1, wherein the tripeptide is XPP, wherein X= C, M, S, or T.

3. Use according to claim 2, wherein the tripeptide is XPP, wherein X= M, S, or T.

4. Use according to any of claims 1-3, wherein the angiotensin-converting enzyme inhibitor is a functional food product.

5. Food product suitable for lowering blood pressure in humans comprising an amount of 5 mg/g or more of XPP, wherein X=C, M, S, T or K.

6. Food product according to claim 5, wherein the amount of XPP is 10mg/g or more.

7. Food product according to claim 5 or 6, wherein the XPP is XPP, wherein X= C, M, S, or T.

8. Food product according to claim 7, wherein the XPP is XPP, wherein X= M, S, or T.

## Patentansprüche

1. Verwendung des Tripeptids XPP, worin X = C, M, S, T oder K, und/oder von Salzen davon für die Herstellung eines Inhibitors des Angiotensin-umwandelnden Enzyms.

2. Verwendung gemäß Anspruch 1, wobei das Tripeptid XPP, worin X = C, M, S oder T, ist.

3. Verwendung gemäß Anspruch 2, wobei das Tripeptid XPP, worin X = M, S oder T, ist.

4. Verwendung gemäß einem der Ansprüche 1-3, wobei der Inhibitor des Angiotensin-umwandelnden Enzyms ein Functional-Food-Produkt ist.

5. Nahrungsmittelprodukt, das zur Senkung des Blutdrucks beim Menschen geeignet ist, umfassend eine Menge von 5 mg/g oder mehr von XPP, worin X = C, M, S, T oder K.

6. Nahrungsmittelprodukt gemäß Anspruch 5, wobei die Menge von XPP 10 mg/g oder mehr ist.

7. Nahrungsmittelprodukt gemäß Anspruch 5 oder 6, wobei das XPP XPP, worin X = C, M, S oder T, ist.

8. Nahrungsmittelprodukt gemäß Anspruch 7, wobei das XPP XPP, worin X = M, S oder T, ist.

**Revendications**

1. Utilisation du tripeptide XPP, où X= C, M, S, T, ou K et/ou des sels de celui-ci pour la préparation d'un inhibiteur de l'enzyme de conversion de l'angiotensine.

2. Utilisation selon la revendication 1, dans laquelle le tripeptide est XPP, où X= C, M, S, ou T.

3. Utilisation selon la revendication 2, dans laquelle le tripeptide est XPP, où X= M, S, ou T.

4. Utilisation selon l'une quelconque des revendications 1-3, dans laquelle l'inhibiteur de l'enzyme de conversion de l'angiotensine est un produit alimentaire fonctionnel.

5. Produit alimentaire approprié pour diminuer la pression sanguine chez les êtres humains comprenant une quantité de 5 mg/g de XPP ou plus, où X= C, M, S, T ou K.

6. Produit alimentaire selon la revendication 5, dans lequel la quantité de XPP est de 10 mg/gou plus.

7. Produit alimentaire selon la revendication 5 ou 6, dans lequel le XPP est XPP, où X= C, M, S, ou T.

8. Produit alimentaire selon la revendication 7, dans lequel le XPP est XPP, où X= M, S, ou T.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0168114 A
- JP 10212245 B
- EP 1231279 A
- WO 0185984 A
- WO 0042066 A

### Non-patent literature cited in the description

- **Gerstein et al.** *The Lancet,* 2000, vol. 355, 253-259
- **Hata, Y et al.** *American Journal of Clinical Nutrition,* 1996, vol. 64, 767-771
- **Y. Poilot et al.** *Journal of Membrane Science,* 1999, vol. 158, 105-114
- **Matsui, T. et al.** *Biosci. Biotech. Biochem.,* 1992, vol. 56, 517-518